# EUROPEAN PATENT APPLICATION

(11) **EP 3 175 769 A2**
(43) Date of publication of application: **07.06.2017**
(21) Application number: 16189758.2
(22) Date of filing: 20.09.2016
(51) Int. Cl.: A61B 1/00, A61B 5/00, A61B 5/06

(54) **ADDING A TRACKING SENSOR TO A RIGID TOOL**

(30) Priority: 21.09.2015 US 201562221367 P; 18.07.2016 US 201615212539
(71) Applicant: Biosense Webster (Israel) Ltd., 2066717 Yokneam (IL)
(72) Inventor: GLINER, Vadim, 2066717 Yokneam (IL)
(74) Representative: Carpmaels & Ransford LLP

(57) **Abstract**

A computerized tomography scan of a patient is registered with a magnetic tracking system configured to track a sensor in proximity to the patient. The sensor is mounted on a probe. When the distal end of the probe is in contact with the patient a vector representing a translation from the sensor to the distal end is determined and used together with the registered image to determine a position of the distal end with respect to the patient.

## Description

### COPYRIGHT NOTICE

A portion of the disclosure of this patent document contains material that is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### CROSS-REFERENCE TO RELATED APPLICATIONS

This Application claims the benefit of U.S. Provisional Application No. 62/221,367, which is herein incorporated by reference. This Application shares disclosure with commonly assigned copending Application Ser. No. 14/986,179, entitled *Adjustable Tracking Sensor Suitable For Different Rigid Tools.*

### BACKGROUND OF THE INVENTION

### 1. Field of the Invention.

This invention relates to instruments for performing medical examinations of the interior of cavities of the body. More particularly, this invention relates to mounting sensors on a probe for determining the position of the probe in the body.

### 2. Description of the Related Art.

In a surgical procedure the distal end of a rigid tool used in the procedure, such as an endoscope, may be tracked by fixedly incorporating a location sensor in the tool. A typical surgical procedure where such a tool is used comprises an investigative ENT (Ear, Nose and Throat) procedure. The sensor may be located at the distal end of the tool; alternatively, the sensor may be located away from the distal end, towards the proximal end of the tool. In the latter case, since the sensor is in a fixed position, compensation for the displacement of the sensor from the distal end may be applied to the sensor's measured location.

However, a number of rigid tools used in surgery do not have location sensors fixedly incorporated into the tool, and while such tools may be tracked by other means, such as fluoroscopy, this type of tracking is typically more inconvenient than tracking using a location sensor on the tool.

### SUMMARY OF THE INVENTION

Embodiments of the present invention overcome the problem associated with tools that do not have sensors incorporated in the tools by providing a sensor that is attached to the tool at any convenient position on the tool. Once attached, the displacement of the attached sensor from the distal end of the tool is automatically measured, and is incorporated into the readings of the attached sensor.

There is provided according to embodiments of the invention a method, which is carried out by receiving a computerized tomography scan of a patient, registering an image of the patient derived from the scan with a magnetic tracking system configured to track a sensor in proximity to the patient, mounting the sensor on a probe, positioning the distal end of the probe in contact with the patient. The method is further carried out by while the distal end is in contact with the patient using data of the registered image to determine a vector representing a translation from the sensor to the distal end, and while tracking the sensor with the magnetic tracking system, adding the vector to a location of the sensor to determine a position of the distal end.

According to a further aspect of the method, mounting the sensor comprises providing a carriage that conforms to a diameter of the probe, placing the sensor on the carriage, and attaching the carriage to the probe.

According to an additional aspect of the method, mounting the sensor is performed by sliding the carriage onto the probe.

According to a further aspect of the method, the carriage has a hole formed therein that is dimensioned to an outer diameter of the probe, and sliding the probe through the hole.

According to yet another aspect of the method, the carriage is fixedly mounted on the probe.

In still another aspect of the method, a calibration includes determining a first vector corresponding to a first translation between a location of the sensor and a point on the longitudinal symmetry axis of the probe, and determining a second vector corresponding to a second translation between the point and the distal end of the probe, and adding the first vector and the second vector to establish a displacement vector.

Another aspect of the method includes determining a length of the second translation by measuring a displacement on the image between the point and another point wherein the other point is determined by detecting a predetermined change in the data of the registered image.

According to still another aspect of the method, the data of the registered image comprise voxel values that indicate radiodensity.

According to yet another aspect of the method, the data of the registered image comprise Hounsfield units and determining a length of the second translation includes measuring the predetermined change in the Hounsfield units.

According to one aspect of the method, registering an image includes disposing a plurality of magnetic field radiators in contact with the patient, radiating alternating magnetic fields at respective frequencies from the magnetic field radiators, detecting the magnetic fields in the sensor, and analyzing the detected magnetic fields to derive the location and an orientation of the sensor with respect to the magnetic field radiators.

In another aspect of the method the magnetic field radiators are mounted on a frame and the frame is placed in contact with the patient.

An additional aspect of the method includes deriving the location and the orientation of the sensor with respect to anatomic features of the patient based on the registered image.

According to a further aspect of the method, registering an image includes receiving a computerized tomographic image of the patient.

There is further provided according to embodiments of the invention an apparatus, including a carriage dimensioned to a diameter of a cylindrical probe and configured to rigidly fasten onto the shaft of the probe, a location sensor disposed in the carriage and responsive to a plurality of magnetic fields at respective frequencies, and a processor operative to compute a position of the distal end of the probe responsively to signals from the location sensor.

Another aspect of the apparatus includes a frame having a plurality of magnetic field radiators mounted thereon for producing the plurality of magnetic fields, wherein the processor is operative to register an image of a patient with the magnetic field radiators.

According to one aspect of the apparatus, the processor is operative for computing the position of the distal end of the probe with respect to coordinates on the registered image.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a schematic illustration of a nasal sinus surgery system, according to an embodiment of the present invention;
Fig. 2 is a schematic illustration of a magnetic field radiation assembly used in the surgery system, according to an embodiment of the present invention;
Fig. 3A is a schematic cross-sectional side view of a probe; Fig. 3B is a schematic cross-sectional front view of the probe; Fig. 3C is a schematic diagram illustrating vectors related to the probe, according to an embodiment of the present invention;
Fig. 4 is a flowchart of steps that are implemented in the operation of the surgery system, according to an embodiment of the present invention; and
Fig. 5 is a schematic illustration of a screen used during implementation of the flowchart, according to an embodiment of the present invention.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily.

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

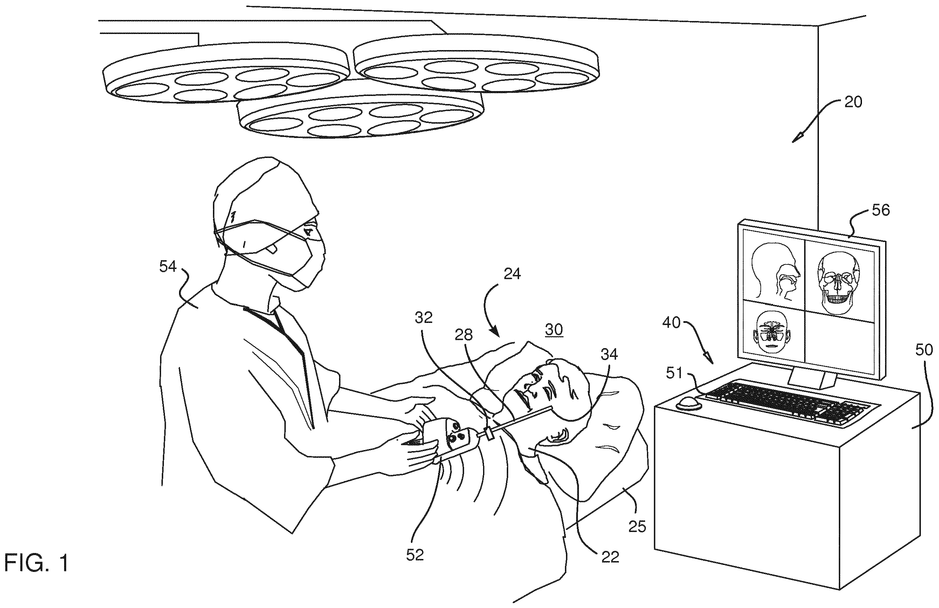

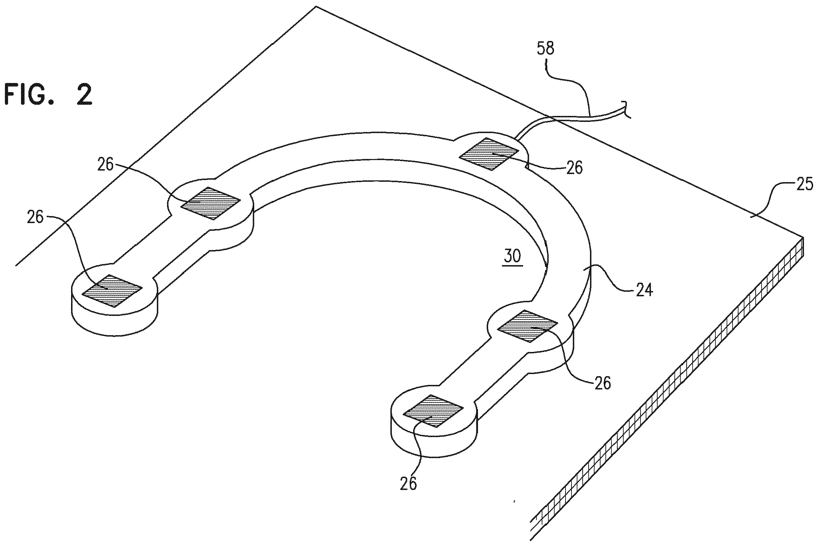

Turning now to the drawings, reference is now made to Fig. 1, which is a schematic illustration of a nasal sinus surgery system 20, and to Fig. 2, which is a schematic illustration of a magnetic field radiation assembly 24 used in the system, according to an embodiment of the present invention. System 20 is typically used during an invasive and/or investigative procedure on a nasal sinus of a patient 22.

For the procedure, assembly 24 may be positioned beneath the head of the patient, for example by fixing the assembly to a bed 25 upon which the patient is lying. Assembly 24 comprises five magnetic field radiators 26, which by way of example are fixed in a horseshoe shaped frame, the frame being positioned beneath the patient so that the radiators surround the head of patient 22. Radiators 26 are configured to radiate alternating magnetic fields at respective frequencies into a region 30, in proximity to assembly 24 and which includes the head of patient 22. The alternating magnetic fields induce signals in a sensor 32, typically a set of three orthogonal coils, and the signals may be analyzed to derive the location and orientation of the sensor with respect to assembly 24. It will be understood that the location and orientation of sensor 32 may be determined for substantially any positioning of the sensor within region 30.

As is described in more detail below, sensor 32 is affixed to a rigid probe 28, and determination of the location and orientation of the sensor enables the location and orientation of a distal end 34 of the probe, that may be inserted into the nasal sinus of the patient, to be tracked. A system using magnetic field radiators, such as radiators 26, for tracking an entity inserted into a patient is described in US Patent Application 14/792,823, to Govari et al., which is incorporated herein by reference. In addition, the Carto^{®} system produced by Biosense Webster, of Diamond Bar, CA, uses a tracking system similar to that described herein for finding the location and orientation of a coil in a region irradiated by magnetic fields.

Elements of system 20, including radiators 26, may be controlled by a system processor 40, comprising a processing unit communicating with one or more memories. Typically the elements may be connected by cables to the processor, for example, radiators 26 may be connected by a cable 58 to processor 40. Alternatively or additionally, the elements may be coupled wirelessly to the processor. Processor 40 may be mounted in a console 50, which comprises operating controls 51 that typically include a keypad and/or a pointing device such as a mouse or trackball. Console 50 also connects to other elements of system 20, such as a proximal end 52 of probe 28. A physician 54 uses the operating controls to interact with the processor while performing the procedure, and the processor may present results produced by system 20 on a screen 56.

Processor 40 uses software stored in a memory of the processor to operate system 20. The software may be downloaded to processor 40 in electronic form, over a network, for example, or it may, alternatively or additionally, be provided and/or stored on non-transitory tangible media, such as magnetic, optical, or electronic memory.

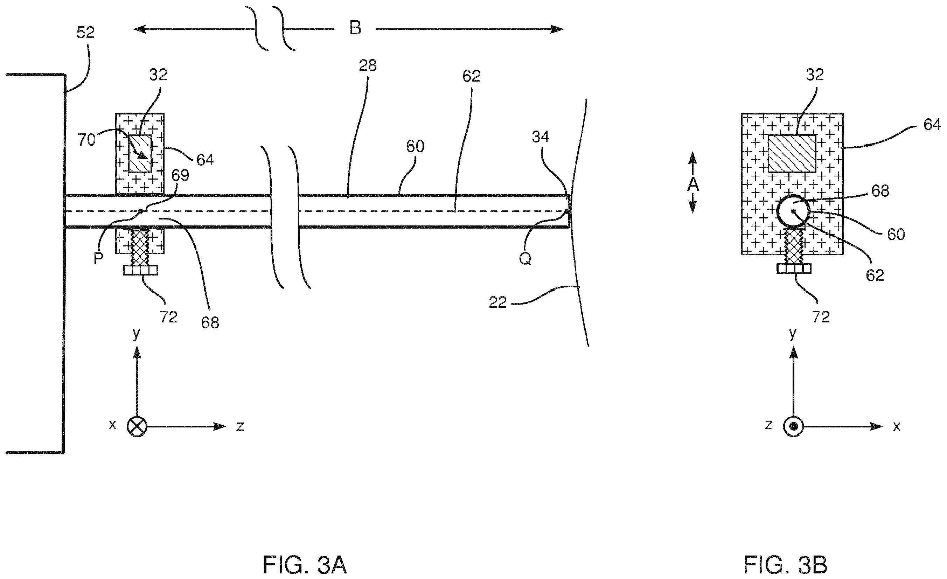

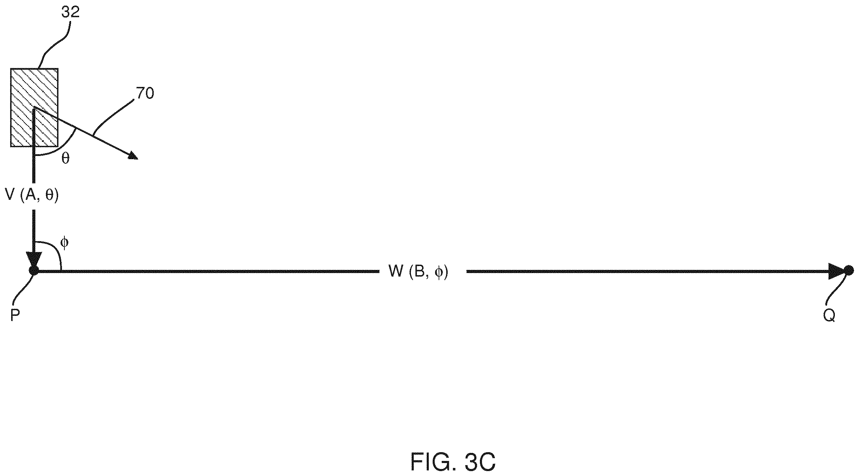

Fig. 3A is a schematic cross-sectional side view of probe 28, Fig. 3B is a schematic cross-sectional front view of the probe, and Fig. 3C is a schematic diagram illustrating vectors related to the probe, according to an embodiment of the present invention. In the following description of probe 28, the probe is assumed to comprise a rigid cylinder 60, having a longitudinal symmetry axis 62. In Figs. 3A and 3B the probe has been drawn on a set of xyz orthogonal axes, with longitudinal symmetry axis 62 defining the z-axis. For clarity, in Figs. 3A and 3B the xyz axes of the probe are drawn displaced from cylinder 60.

Sensor 32 is fixed to cylinder 60 by a sensor holder 64, which is typically formed from a plastic so as to completely encapsulate the sensor. As explained herein, signals from the sensor, generated in response to the magnetic fields interacting with the sensor, are used to determine a location and an orientation of the sensor. Conducting wires that convey the signals from the sensor may be connected to proximal end 52 of probe 28, and from there to console 50. The conducting wires are not shown in Figs. 3A and 3B.

The sensor is assumed to have a sensor direction 70, typically, but not necessarily, the direction of an internal axis of symmetry of the sensor, and the orientation referred to herein measures the orientation of the sensor direction with respect to a frame of reference defined by radiators 26. Sensor direction 70 of sensor 32 is shown schematically in Figs. 3A and 3C as an arrow.

Sensor holder 64 is produced to have a hole 68, which is formed to have a diameter substantially the same as that of cylinder 60, but sufficiently different so that there is a sliding fit between the holder and cylinder 60. When holder 64 is produced, a center of hole 68 is made to be a known distance A from sensor 32. A typical value of A is 0.5 cm, but A may be smaller or larger than this value. A series of sensor holders may be constructed, having holes 68 that are dimensioned to tools having different diameters. In addition, by virtue of being comprised in holder 64, the center of hole 68 has a known orientation θ with respect to sensor direction 70. There is thus a known displacement vector (A, θ), herein also termed vector V, from sensor 32 to the center of hole 68, as shown in Fig. 3C.

Hole 68 has an axis of symmetry 69 that is typically orthogonal to vector V, and which by virtue of being formed when holder 64 is produced, has a known direction φ with respect to vector V (Fig. 3C).

As is also described below, in operating system 20 hole 68 of sensor holder 64 is slid onto cylinder 60, and the holder is fixed to the cylinder when the holder is close to proximal end 52. It will be understood that in sliding cylinder 60 within hole 68 axes 69 and 62 are coincident, and also coincide with direction φ. Holder 64 comprises a setscrew 72, having a head, which may be grasped by physician 54. Using the head the professional is able to hand-tighten the setscrew to fix holder 64 at a desired position along cylinder 60. The distance from the center of sensor 32 to distal end 34 is assumed to be a distance B. Unlike distance A, distance B is not known when sensor holder 64 is fixed to cylinder 60, but as is described below, in operation of system 20 processor 40 is able to calculate distance B.

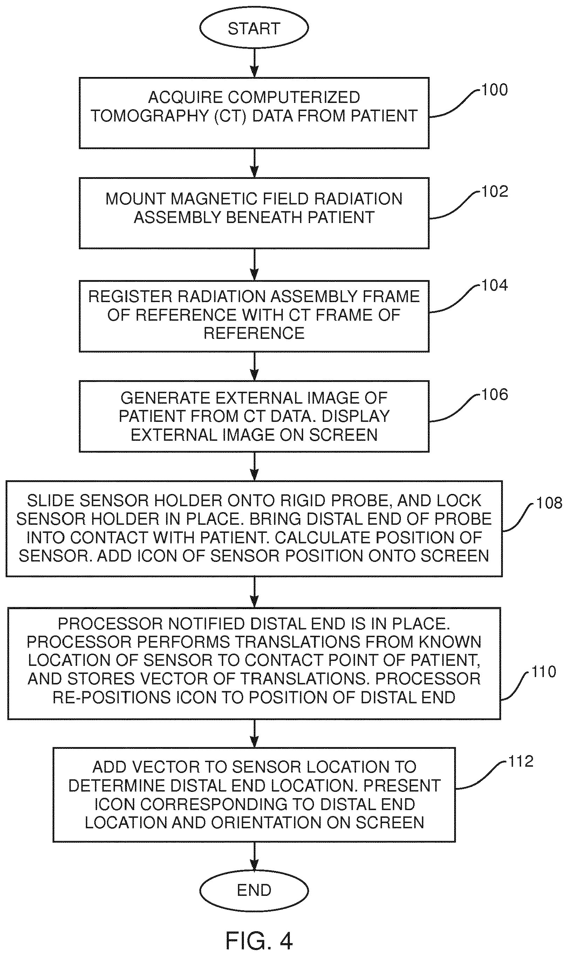

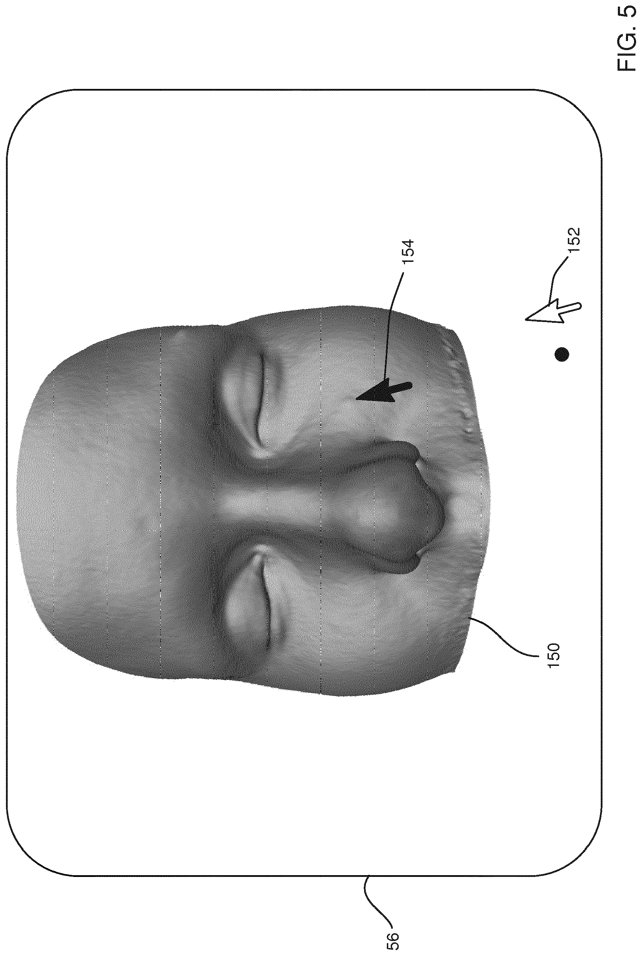

Fig. 4 is a flowchart of steps that are implemented in the operation of system 20, and Fig. 5 is a schematic illustration of screen 56 during implementation of the flowchart, according to an embodiment of the present invention. The steps of the flowchart are also illustrated by Figs. 3A, 3B, and 3C.

In an initial step 100, the head of patient 22 is scanned by computerized tomography (CT), herein by way of example assumed to be fluoroscopic CT, and the CT data from the scan is acquired by processor 40. The CT scan of patient 22 may be performed independently of the implementation of the remaining steps of the flowchart, which correspond to the sinus surgery procedure. Typically, step 100 may be performed a number of days before the following surgery steps of the procedure.

In a first procedure step 102, radiation assembly 24 is mounted beneath the head of patient 22. Radiators 26 are then operated, and in a registration step 104 a frame of reference of the radiators is registered with the frame of reference of the subject's head. The registration is typically by any means known in the art, e.g., by placing a magnetic field sensor coil, or a grouping of such coils, in one or more known locations and orientations with respect to the external features of the patient as well as with the frame holding the radiators.

In an initial display step 106, processor 40 generates a representation 150, also referred to herein as image 150, of external features of the patient, using the CT data received in step 100. The CT data is in the form of voxels with Hounsfield units (HU), and it will be appreciated that image 150 of the external features of patient 22 can be generated from voxel values and their HU values. Processor 40 displays image 150 on screen 56, and Fig. 5 schematically illustrates the image as displayed on the screen.

In an operation step 108, the physician slides hole 68 of sensor holder 64 onto rigid cylinder 60 of probe 28, and the physician then uses setscrew 72 to lock the sensor holder in place, near proximal end 52 of the probe. Once the holder is locked in place, the physician brings distal end 34 of the probe into contact with a selected region of the external features of the patient, for example a region at the side of the patient's nose.

The positioning of the distal end of necessity brings sensor holder 64 and its encapsulated sensor 32 into region 30 (Figs. 1 and 2), so that processor 40 is able to calculate the location and orientation of the sensor. Once the processor has performed this calculation, it typically introduces an icon 152, representative of sensor direction 70, onto screen 56, in proximity to image 150. Icon 152 is located and orientated on screen 56 in accordance with the location and orientation of sensor 32, determined from the sensor signals, within the common frame of reference of image 150 and radiators 26.

By virtue of the fact that the physician is holding probe 28, the physician is aware of the actual location and orientation of sensor 32. Comparison of the location and orientation of icon 152 with the actual location and orientation of sensor 32 provides confirmation to the physician of the correct operation of system 20.

In a calibration step 110 the physician notifies processor 40 that the distal end of the probe is in contact with an external feature of the patient, typically by using controls 51. On receipt of the notification, the processor performs two translations on the known location of sensor 32. A first translation corresponds to vector V (A, θ), (Fig. 3C) so that the processor translates the location of the sensor by a value A along a direction defined by θ to a point P on axis 62 (Fig. 3A). A point P', corresponding to point P, is drawn in Fig. 5, to illustrate the termination of the first translation. Typically, point P' is not drawn on screen 56.

From point P the processor performs a second translation, in a direction corresponding to direction φ. Since axes 69 and 62 are coincident, the second translation is in a direction corresponding to translating along axis 62. The processor uses the data for image 150 to determine the actual length of the second translation, by determining from the image data where point P, moving in direction φ along axis 69, meets an external surface of patient 22. The meeting with the external surface occurs when there is at least a predetermined change in radiodensity as measured in the image, e.g., a change in the value of the Hounsfield units of the image data. Suitable values for the change are 200 - 500 Hounsfield units. The meeting is assumed to be at a point Q on axis 62. Point Q is at a distance B, now known, from point P, and the second translation thus corresponds to a vector (B, φ), herein also termed vector W, and illustrated in Fig. 3C.

It will be understood that even though the calculation of the position of point Q uses CT image data, since the image is registered with the actual external features of patient 22, point Q corresponds with an actual external point of the patient.

At the conclusion of the calibration step the processor deletes icon 152 from screen 56, and positions an icon 154 at a position of image 150 corresponding to point Q. Comparison of the location and orientation of icon 154 with the actual location and orientation of distal end 34 provides confirmation to the physician of the correct completion of the calibration step.

The sum of the two translations, V + W, of the calibration step is a vector that is stored by processor 40.

In a continuing tracking step 112, the processor adds the vector stored in step 110 to the location of the sensor in order to determine the location of distal end 34. The orientation of the distal end corresponds to direction φ, which is also determined by the processor in tracking the sensor. Thus the processor is able to calculate the location and orientation of distal end 34 by determining the location and orientation of sensor 32. The processor may position an icon corresponding to the location and orientation of the distal end on screen 56. In some embodiments, if the distal end is within patient 22, the external features of image 150 that may obscure the icon are rendered at least partially transparent. The position of the distal end with respect to anatomic features of the patient may be derived based on the calculated position of the distal end with respect to coordinates on the registered image.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. A method, comprising the steps of:
receiving a computerized tomography scan of a patient;
registering an image of the patient derived from the scan with a magnetic tracking system configured to track a sensor in proximity to the patient;
mounting the sensor on a probe having a distal end;
positioning the distal end in contact with the patient;
while the distal end is in contact with the patient, using data of the registered image to determine a displacement vector representing a translation from the sensor to the distal end; and
while tracking the sensor with the magnetic tracking system, adding the displacement vector to a location of the sensor to determine a position of the distal end.

2. The method according to claim 1, wherein mounting the sensor comprises:
providing a carriage that conforms to a diameter of the probe;
placing the sensor on the carriage; and
attaching the carriage to the probe.

3. The method according to claim 2, wherein mounting the sensor comprises sliding the carriage onto the probe.

4. The method according to claim 3, wherein the carriage has a hole formed therein that is dimensioned to an outer diameter of the probe, and wherein sliding the carriage comprises sliding the probe through the hole.

5. The method according to claim 2, wherein mounting the sensor comprises fixedly mounting the carriage onto the probe.

6. The method according to claim 1, wherein the probe has a longitudinal symmetry axis, further comprising performing a calibration comprising:
determining a first vector corresponding to a first translation between a location of the sensor and a point on the longitudinal symmetry axis of the probe; and
determining a second vector corresponding to a second translation between the point and the distal end of the probe; and
adding the first vector and the second vector to establish the displacement vector.

7. The method according to claim 6, further comprising determining a length of the second translation by measuring a displacement on the image between the point and another point wherein the other point is determined by detecting a predetermined change in the data of the registered image.

8. The method according to claim 7, wherein the data of the registered image comprise voxel values that indicate radiodensity.

9. The method according to claim 7, wherein the data of the registered image comprise Hounsfield units and determining a length of the second translation comprises measuring the predetermined change in the Hounsfield units.

10. The method according to claim 1, wherein registering an image comprises:
disposing a plurality of magnetic field radiators in relation to the patient;
radiating alternating magnetic fields at respective frequencies from the magnetic field radiators;
detecting the magnetic fields in the sensor; and
analyzing the detected magnetic fields to derive the location and an orientation of the sensor with respect to the magnetic field radiators.

11. The method according to claim 10, wherein disposing a plurality of magnetic field radiators comprises the steps of:
mounting the magnetic field radiators on a frame; and
placing the frame in contact with the patient.

12. The method according to claim 10, further comprising deriving the location and the orientation of the sensor with respect to anatomic features of the patient based on the registered image.

13. The method according to claim 1, wherein registering an image comprises receiving a computerized tomographic image of the patient.

14. An apparatus, comprising;
a carriage dimensioned to a diameter of a cylindrical probe having a shaft and a distal end, and configured to rigidly fasten onto the shaft;
a location sensor disposed in the carriage and responsive to a plurality of magnetic fields at respective frequencies; and
a processor operative to compute a position of the distal end of the probe responsively to signals from the location sensor.

15. The apparatus according to claim 14, further comprising a frame having a plurality of magnetic field radiators mounted thereon for producing the plurality of magnetic fields, wherein the processor is operative to register an image of a patient with the magnetic field radiators.

16. The apparatus according to claim 15, wherein the processor is operative for computing the position of the distal end of the probe with respect to coordinates on the registered image.
